# EUROPEAN PATENT SPECIFICATION

(11) **EP 4 425 502 B1**
(45) Date of publication and mention of the grant of the patent: **12.11.2025**
(21) Application number: 23823835.6
(22) Date of filing: 08.06.2023
(51) Int. Cl.: G16C 20/40, G16C 20/30, G06F 30/27, G06N 20/00, G16C 60/00, G16C 20/70

(54) **THERMOSETTING EPOXY RESIN COMPOSITION SEARCH METHOD, INFORMATION PROCESSING DEVICE, AND PROGRAM**
SUCHVERFAHREN FÜR WÄRMEHÄRTENDE EPOXIDHARZZUSAMMENSETZUNG, INFORMATIONSVERARBEITUNGSVORRICHTUNG UND PROGRAMM
PROCÉDÉ DE RECHERCHE DE COMPOSITION DE RÉSINE ÉPOXY THERMODURCISSABLE, DISPOSITIF DE TRAITEMENT D'INFORMATIONS ET PROGRAMME

(30) Priority: 15.06.2022 JP 2022096819
(43) Date of publication of application: 04.09.2024
(73) Proprietor: DIC Corporation, Tokyo 174-8520 (JP)
(72) Inventor: NISHIYAMA Yutaro, Ichihara-shi, Chiba 290-8585 (JP); IMADA Tomoyuki, Ichihara-shi, Chiba 290-8585 (JP)
(74) Representative: Scott, Stephen John
(86) International application number: PCT/JP2023/021421
(87) International publication number: WO 2023/243544

(56) References cited:
- WO-A1-2020/152993
- WO-A1-2021/079985
- WO-A1-2022/080443
- JP-A- 2020 030 638
- JP-A- 2021 038 344
- JP-A- 2022 516 697
- JP-B2- 6 832 678
- JIN KAI ET AL: "Composition optimization of a high-performance epoxy resin based on molecular dynamics and machine learning", vol. 194, 1 September 2020 (2020-09-01), AMSTERDAM, NL, pages 108932, XP093200998, ISSN: 0264-1275, Retrieved from the Internet <URL:https://pdf.sciencedirectassets.com/313059/1-s2.0-S0264127520X00074/1-s2.0-S0264127520304664/main.pdf?X-Amz-Security-Token=IQoJb3JpZ2luX2VjEJH//////////wEaCXVzLWVhc3QtMSJGMEQCIGLZz8FQ3B+vilsaYfyGm95aL70h6PpM4fCbfeVPd711AiBn66fgv/a8ZkoHCwn1L1FxIqCZ4ZvYukCXTrvXbaTmliq6BQiq//////////8BEAUaDDA1OTAwMzU0N> DOI: 10.1016/j.matdes.2020.108932
- NAKAJIN KOKIN ET AL: "Prediction of physical properties of thermosetting resin by using machine learning and structural formulas of raw materials", vol. 5, no. 29-30, 1 June 2020 (2020-06-01), pages 1567 - 1575, XP093201337, ISSN: 0272-9172, Retrieved from the Internet <URL:http://link.springer.com/content/pdf/10.1557/adv.2020.266.pdf> DOI: 10.1557/adv.2020.266
- AMAMOTO YOSHIFUMI: "Data-driven approaches for structure-property relationships in polymer science for prediction and understanding", POLYMER JOURNAL, NATURE PUBLISHING GROUP UK, LONDON, vol. 54, no. 8, 3 May 2022 (2022-05-03), pages 957 - 967, XP037903421, ISSN: 0032-3896, [retrieved on 20220503], DOI: 10.1038/S41428-022-00648-6
- LIU BEI ET AL: "Performance optimization of shape memory epoxy polymers based on machine learning", vol. 33, no. 4, 28 December 2021 (2021-12-28), GB, pages 1222 - 1232, XP093242919, ISSN: 1042-7147, Retrieved from the Internet <URL:https://onlinelibrary.wiley.com/doi/full-xml/10.1002/pat.5595> DOI: 10.1002/pat.5595

## Description

### Technical Field

The present disclosure relates to a method of searching for a thermosetting epoxy resin composition, an information processor, and a program. The present application claims the priority of Japanese Patent Application No. 2022-096819 filed on June 15, 2022 in Japan.

### Background Art

There have been known composite materials, such as carbon fiber reinforced plastics (hereinafter also referred to as CFRP) produced by impregnating carbon fibers with a composition containing a thermosetting epoxy resin or the like (hereinafter also referred to as a thermosetting epoxy resin composition) and heat-curing the composition. CFRP is a fiber-reinforced composite material containing a plastic as a base material and carbon fibers as reinforcing fibers. CFRP is a high-performance material that is lighter in weight than general structural metallic materials, has high specific strength and specific rigidity, and has good electrical characteristics and high corrosion resistance. Thus, in recent years, CFRP has been used as a material replacing metallic materials in a wide range of applications, such as structural members of aerospace structures, automobiles, two-wheeled vehicles, and ships, large-sized industrial machine parts, and sporting goods. Thermosetting epoxy resin compositions used as raw materials for composite materials, such as CFRP, are required to have characteristics, such as strength and heat resistance, depending on the use of the composite materials. However, some of these characteristics of thermosetting epoxy resin compositions may be contradictory to each other, and it is not necessarily easy to search for a thermosetting epoxy resin composition with required characteristics in a well-balanced manner. Composite materials, such as CFRP, have a wide range of applications as described above, and it is therefore desired to diversify and improve the performance by optimizing the blending of raw materials.

On the other hand, there is a known technique of modeling a material model, learning the material model by machine learning, and then using the learned material model to search for a structure of a novel material from target physical property information (for example, Patent Literature 1).

Another known technique of modeling a material is disclosed in Non-Patent Literature 1, which discloses a neural network model comprising an input layer, three hidden layers, and an output layer, in which DGEBA, TGDDM, DICY, DDS, PES, and PEI are selected as input vectors and the glass transition temperature (Tg), Young's modulus (E), the ultimate tensile strength (UTS), and elongation (δ) are selected as output vectors.

### Citation List

### Patent Literature

PTL 1: Japanese Patent No. 6832678

### Non-Patent Literature

NPL 1: Jin Kai ET AL: "Composition optimization of a high-performance epoxy resin based on molecular dynamics and machine learning", Materials & Design, vol. 194, 1 September 2020 (2020-09-01), page 108932, AMSTERDAM, NL, ISSN: 0264-1275, DOI: 10.1016/j.matdes.2020.108932

### Summary of Invention

### Technical Problem

The technique of searching for a material described in Patent Literature 1 is directed to a general material model, and searching for a thermosetting epoxy resin composition and optimizing the blending of raw materials of a composite material have not been particularly considered in the related art.

An object of the present disclosure made in view of such circumstances is to improve a technique of searching for a thermosetting epoxy resin composition.

### Solution to Problem

A method of searching for a thermosetting epoxy resin composition according to an embodiment of the present disclosure is
a method of searching for a thermosetting epoxy resin composition executed by an information processor, the method including the steps of:
calculating a feature value on the basis of actual data related to a thermosetting epoxy resin composition, using the feature value as an explanatory variable of a plurality of predictive models, and training a plurality of predictive models each corresponding to a target variable using the actual data, wherein the feature value includes Log P related to a thermosetting epoxy resin composition; and
searching for a thermosetting epoxy resin composition with a desired balance of physical properties by inverse analysis using the plurality of predictive models,
wherein the actual data include a blend raw material and a blend ratio of the thermosetting epoxy resin composition, a polymer composition related to the blend raw material, and a structural formula related to the blend raw material, and
the target variables include tensile strength, fracture toughness, and a glass transition temperature.

In a search method according to an embodiment of the present disclosure,
the plurality of predictive models include a predictive model to which the tensile strength is input, a predictive model to which the fracture toughness is input, and a predictive model to which the glass transition temperature is input.

In a search method according to an embodiment of the present disclosure,
the feature value further includes at least one of a molecular fingerprint and a molecular descriptor.

In a search method according to an embodiment of the present disclosure,
the actual data include actual data of a thermosetting epoxy resin composition used for a predetermined application and actual data of a thermosetting epoxy resin composition used for an application other than the predetermined application, and
the step of creating the plurality of predictive models includes
creating the plurality of predictive models using the actual data of the thermosetting epoxy resin composition used for the application other than the predetermined application and then relearning the plurality of predictive models using the actual data of the thermosetting epoxy resin composition used for the predetermined application.

An information processor according to an embodiment of the present disclosure is
an information processor that includes a control unit and searches for a thermosetting epoxy resin composition, wherein
the control unit is configured to
calculate a feature value on the basis of actual data related to a thermosetting epoxy resin composition, use the feature value as an explanatory variable of a plurality of predictive models, and train a plurality of predictive models each corresponding to a target variable using the actual data, wherein the feature value includes Log P related to a thermosetting epoxy resin composition, and
search for a thermosetting epoxy resin composition with a desired balance of physical properties by inverse analysis using the plurality of predictive models,
wherein the actual data include a blend raw material and a blend ratio of the thermosetting epoxy resin composition, a polymer composition related to the blend raw material, and a structural formula related to the blend raw material, and
the target variables include tensile strength, fracture toughness, and a glass transition temperature.

A non-transitory computer-readable recording medium according to an embodiment of the present disclosure is
a non-transitory computer-readable recording medium storing instructions that, when executed by a processor, causes the processor
to calculate a feature value on the basis of the actual data related to a thermosetting epoxy resin composition, to use the feature value as an explanatory variable of a plurality of predictive models, and to train the plurality of predictive models each corresponding to a target variable using the actual data, wherein the feature value includes Log P related to a thermosetting epoxy resin composition; and
to search for a thermosetting epoxy resin composition with a desired balance of physical properties by inverse analysis using the plurality of predictive models,
wherein the actual data include a polymer composition, a reaction solvent, and a reaction parameter related to the thermosetting epoxy resin composition,
the actual data include a blend raw material and a blend ratio of the thermosetting epoxy resin composition, a polymer composition related to the blend raw material, and a structural formula related to the blend raw material, and
the target variables include tensile strength, fracture toughness, and a glass transition temperature.

### Advantageous Effects of Invention

A method of searching for a thermosetting epoxy resin composition, an information processor, and a program according to an embodiment of the present disclosure can improve a technique of searching for a thermosetting epoxy resin composition.

### Brief Description of Drawings

[Fig. 1] Fig. 1 is a diagram of an overview of an embodiment of the present disclosure.
[Fig. 2] Fig. 2 is a block diagram of a schematic configuration of an information processor that searches for a thermosetting epoxy resin composition according to an embodiment of the present disclosure.
[Fig. 3] Fig. 3 is a flow chart of an operation of a learning process of an information processor that searches for a thermosetting epoxy resin composition according to an embodiment of the present disclosure.
[Fig. 4] Fig. 4 is a flow chart of an operation of a search process of an information processor that searches for a thermosetting epoxy resin composition according to an embodiment of the present disclosure.

### Description of Embodiments

A method of searching for a material according to an embodiment of the present disclosure is described below with reference to the accompanying drawings.

In the drawings, the same or equivalent parts are denoted by the same reference numerals and letters. In the description of the present embodiment, description of the same or equivalent parts is omitted or simplified as appropriate.

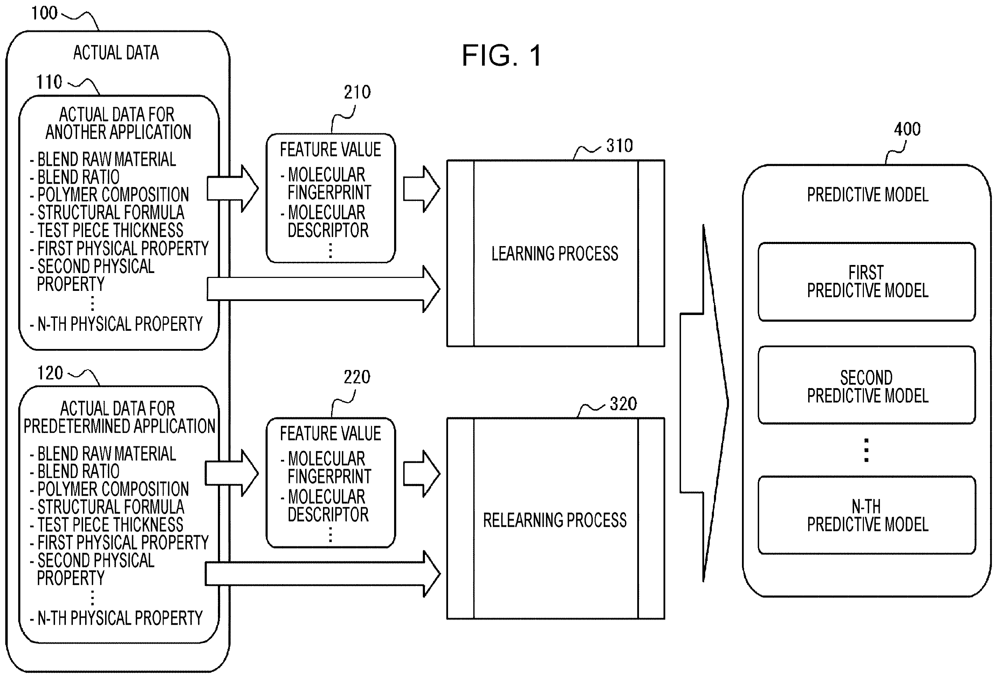

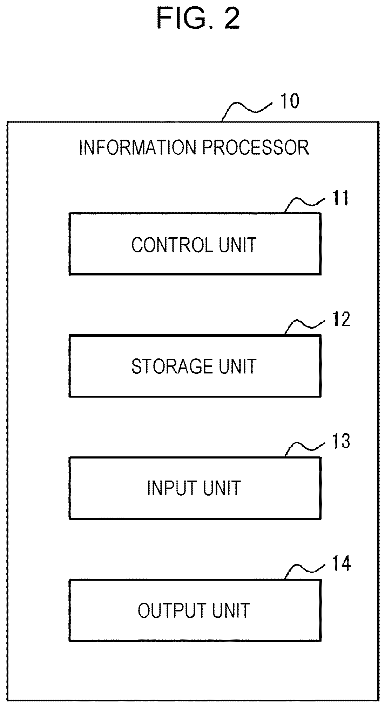

A method of searching for a thermosetting epoxy resin composition according to the present embodiment is outlined below with reference to Figs. 1 and 2.

First, the present embodiment is outlined below. Actual data 100 shown in Fig. 1 are used in the method of searching for a thermosetting epoxy resin composition according to the present embodiment. An information processor 10 shown in Fig. 2 executes the method of searching for a thermosetting epoxy resin composition according to the present embodiment. The information processor 10 trains a plurality of predictive models 400 each corresponding to a target variable using the actual data 100 related to a thermosetting epoxy resin composition.

The actual data 100 include actual data 110 for another application and actual data 120 for a predetermined application. The actual data 120 for the predetermined application are, for example, actual data related to a thermosetting epoxy resin composition for a composite material. More specifically, the actual data 120 include, for example, actual data related to a thermosetting epoxy resin composition used for CFRP. The actual data 110 for another application are actual data of a thermosetting epoxy resin composition used for an application other than the predetermined application (here, an application other than the composite material). Each of the actual data 110 for another application and the actual data 120 for the predetermined application includes a blend raw material and a blend ratio related to the thermosetting epoxy resin composition, a polymer composition of the blend raw material, a structural formula of the blend raw material, a test piece thickness, and first to N-th physical properties. The blend raw material contains a thermosetting epoxy resin, a toughness improver, a curing agent, and a curing catalyst. The thermosetting epoxy resin, the toughness improver, and the curing agent, and the curing catalyst can be selected from a plurality of types. For example, the blend raw material may be selected from a total of 35 types including 19 types of thermosetting epoxy resins, 5 types of toughness improvers, 3 types of curing agents, and 8 types of curing catalysts. The blend ratio is a weight ratio of blend raw materials and includes, for example, a weight ratio of the curing agent to the thermosetting epoxy resin. The curing agent is, for example, an amine, and in the following description of the present embodiment the curing agent is assumed to be an amine. The test piece thickness is the thickness of a test piece in a physical property measurement and is, for example, 2 mm or 4 mm.

The first to N-th physical properties correspond to a plurality of target variables. N is a positive integer. The information processor 10 trains first to N-th predictive models, each corresponding to one of N target variables. The plurality of target variables include contradictory characteristics. For example, the plurality of target variables include tensile strength, fracture toughness, and heat resistance. An example of the heat resistance may be a glass transition temperature (a temperature with the maximum change in elastic modulus in measurement with a viscoelastometer). In the present embodiment, a glass transition temperature is used as a measure of heat resistance in an example described below. Among these physical properties, fracture toughness and tensile strength are contradictory characteristics. Fracture toughness and glass transition temperature are also contradictory characteristics.

First, the information processor 10 performs a learning process 310 of the predictive models 400 using the actual data 110 for another application. The information processor 10 calculates a feature value 210 from the actual data 110 for another application. The information processor 10 trains the predictive models 400 using the feature value 210 as an explanatory variable and each physical property as a target variable. More specifically, the information processor 10 trains a first predictive model using the feature value 210 as an explanatory variable and a first physical property as a target variable. The information processor 10 trains a second predictive model using the feature value 210 as an explanatory variable and a second physical property as a target variable. Likewise, the information processor 10 trains the N-th predictive model using the feature value 210 as an explanatory variable and an N-th physical property as a target variable. The feature value 210 used for the learning process of the first to N-th predictive models may be appropriately selected according to the relationship between each feature value and the target variable. In other words, the feature value 210 used for learning of the first to N-th predictive models may be different from each other.

Next, the information processor 10 performs a relearning process 320 of the predictive models 400 using the actual data 120 for the predetermined application. First, the information processor 10 calculates a feature value 220 from the actual data 120 for the predetermined application. The information processor 10 performs a relearning process of each predictive model using the feature value 220 as an explanatory variable and each physical property as a target variable. More specifically, the information processor 10 performs a relearning process of the first predictive model using the feature value 220 as an explanatory variable and the first physical property as a target variable. The information processor 10 performs a relearning process of the second predictive model using the feature value 220 as an explanatory variable and the second physical property as a target variable. The information processor 10 performs a relearning process of the N-th predictive model using the feature value 220 as an explanatory variable and the N-th physical property as a target variable. The feature value 220 used for the relearning process of the first to N-th predictive models may be appropriately selected according to the relationship between each feature value and the target variable. In other words, the feature value 220 used for learning of the first to N-th predictive models may be different from each other. The information processor 10 searches for a thermosetting epoxy resin composition with a desired balance of physical properties by inverse analysis using the first to N-th predictive models learned in this manner.

As described above, in the present embodiment, a plurality of predictive models are trained on the basis of actual data related to a thermosetting epoxy resin composition. A thermosetting epoxy resin composition with a desired balance of physical properties is searched for by inverse analysis using the plurality of predictive models. Thus, the search technique is improved in that a thermosetting epoxy resin composition with a desired balance of characteristics can be searched for.

### (Configuration of Information Processor)

Next, each component of the information processor 10 is described in detail below. The information processor 10 is an apparatus used by a user. For example, a personal computer, a server computer, a general-purpose electronic device, or a dedicated electronic device can be adopted as the information processor 10.

As shown in Fig. 2, the information processor 10 includes a control unit 11, a storage unit 12, an input unit 13, and an output unit 14.

The control unit 11 includes at least one processor, at least one dedicated circuit, or a combination thereof. The processor is a general-purpose processor, such as a central processing unit (CPU) or a graphics processing unit (GPU), or a dedicated processor specialized for specific processing. The dedicated circuit is, for example, a field-programmable gate array (FPGA) or an application specific integrated circuit (ASIC). The control unit 11 executes processing related to the operation of the information processor 10 while controlling each unit of the information processor 10.

The storage unit 12 includes at least one semiconductor memory, at least one magnetic memory, at least one optical memory, or a combination of at least two of these memories. The semiconductor memory is, for example, a random access memory (RAM) or a read only memory (ROM). The RAM is, for example, a static random access memory (SRAM) or a dynamic random access memory (DRAM). The ROM is, for example, an electrically erasable programmable read only memory (EEPROM). The storage unit 12 functions as, for example, a main storage, an auxiliary storage, or a cache memory. The storage unit 12 stores data used for the operation of the information processor 10 and data obtained by the operation of the information processor 10.

The input unit 13 includes at least one input interface. The input interface is, for example, a physical key, a capacitance key, a pointing device, or a touch screen integrated with a display. The input interface may be, for example, a microphone that receives voice input or a camera that receives gesture input. The input unit 13 receives an operation of inputting data used for the operation of the information processor 10. Instead of being included in the information processor 10, the input unit 13 may be connected to the information processor 10 as an external input device. The connection method can be, for example, a universal serial bus (USB), a high-definition multimedia interface (HDMI) (registered trademark), Bluetooth (registered trademark), or the like.

The output unit 14 includes at least one output interface. The output interface is, for example, a display that outputs information in the form of an image. The display is, for example, a liquid crystal display (LCD) or an organic electro luminescence (EL) display. The output unit 14 displays and outputs data obtained by the operation of the information processor 10. Instead of being included in the information processor 10, the output unit 14 may be connected to the information processor 10 as an external output device. The connection method can be, for example, USB, HDMI (registered trademark), Bluetooth (registered trademark), or the like.

The functions of the information processor 10 are implemented by a processor corresponding to the information processor 10 executing a program according to the present embodiment. That is, the functions of the information processor 10 are implemented by software. The program causes a computer to execute the operation of the information processor 10 and thereby causes the computer to function as the information processor 10. In other words, a computer executes the operation of the information processor 10 in accordance with the program and thereby functions as the information processor 10.

In the present embodiment, the program can be recorded in a computer-readable recording medium. The computer-readable recording medium includes a non-transitory computer-readable medium, for example, a magnetic recording apparatus, an optical disk, a magneto-optical recording medium, or a semiconductor memory. The program is distributed, for example, by selling, transferring, or lending a portable recording medium, such as a digital versatile disc (DVD) or a compact disc read only memory (CD-ROM), in which the program is recorded. The program may also be distributed by storing the program in a storage of an external server and transmitting the program from the external server to another computer. The program may also be provided as a program product.

A part or all of the functions of the information processor 10 may be realized by a dedicated circuit corresponding to the control unit 11. In other words, a part or all of the functions of the information processor 10 may be realized by hardware.

In the present embodiment, the storage unit 12 stores the actual data 100, the feature values 210 and 220, and the predictive models 400. The feature values 210 and 220 are calculated on the basis of the actual data 100. In the present embodiment, the feature values can be calculated by various methods. For example, the feature values 210 and 220 may be calculated by computer processing based on at least one characteristic data among the actual data 100. The feature values 210 and 220 may also be determined by selecting characteristic data from the actual data 100. In other words, the phrase "calculating a feature value based on actual data", as used herein, refers to at least one of calculation based on the actual data and determination based on the actual data.

The feature value 210 may include any data that represent a feature of a thermosetting epoxy resin composition. For example, the feature value 210 may include a blend raw material and a blend ratio related to a thermosetting epoxy resin composition. For example, the feature value 210 may include at least one of a molecular fingerprint and a molecular descriptor. The molecular descriptor can be obtained from the information of a blend raw material (a thermosetting epoxy resin, a toughness improver, a curing agent, a curing catalyst, or the like) represented by SMILES notation using RDKit, which is a publicly known molecular descriptor generation library, alvaDesc, which is commercially available software, or the like. More specifically, the molecular descriptors of the blend raw materials are obtained and then weight-averaged on the basis of the blend ratio of the blend raw materials to obtain the molecular descriptor of the thermosetting epoxy resin composition. Such a molecular descriptor may be a zero-dimensional descriptor, such as the molecular weight, the number of atoms in a molecule, or the number of bonds. It may be a one-dimensional descriptor, such as the number of functional groups in a molecule, the number of alicyclic structures, or the number of aromatic rings. It may be a two-dimensional descriptor, such as a topological descriptor representing a molecule based on a graph theory, such as a Wiener index, a Chi connectivity index, a Balaban index, or a BCUT descriptor. It may be a three-dimensional descriptor, such as a descriptor calculated on the basis of the three-dimensional structure of a molecule, such as 3D-MoRSE, WHIM, CoMFA, or CoMSIA. It may be a four-dimensional descriptor, such as a descriptor calculated in consideration of interaction, such as Hopfinger, which can be appropriately selected and used. Among them, it is, for example, MaxPartialCharge, MinPartialCharge, MaxEStateIndex, MinEStateIndex, QED, MolMR, MolWt, ExactMolWt, HeavyAtomCount, HeavyAtomMolWt, NHOHCount, NOCount, NumHAcceptors, NumHDonors, NumHeteroatoms, NumRotatableBonds, NumValenceElectrons, NumAmideBonds, NumAromaticRings, NumSaturatedRings, NumAliphaticRings, NumAromaticHeterocycles, NumSaturatedHeterocycles, NumAliphaticHeterocycles, NumAromaticCarbocycles, NumSaturatedCarbocycles, NumAliphaticCarbocycles, RingCount, FractionCSP3, NumSpiroAtoms, NumBridgeheadAtoms, TPSA, LabuteASA, PEOE_VSA1 to PEOE_VSA14, SMR_VSA1 to SMR_VSA10, SlogP_VSA1 to SlogP_VSA12, EState_VSA1 to EState_VSA11, VSA_EState1 to VSA_EState10, BCUT2D, BalabanJ, BertzCT, Ipc, HallKierAlpha, Kappa1-Kappa3, Phi, Chi0, Chi1, Chi0n to Chi4n, or Chi0v to Chi4v. Other useful descriptors generated can be appropriately used. Log P is the logarithm of the partition coefficient (Po/w) of a material in a 1-octanol/water two-phase solvent. In other words, Log P is an index value indicating the degree of hydrophobicity or hydrophilicity of a thermosetting epoxy resin composition. The value may be calculated from a value measured by a flask shaking method or an HPLC method or from a molecular structure by CLogP (calculated by a calculation method of Leo, Hansch, et al.), MiLogP (calculated by a calculation method of Molinspiration), MolLogP (calculated by a calculation method of Crippen), SLogP (calculated by a calculation method of CCG), ALogPS (calculated by a calculation method of Tetko, et al.), or the like. Another useful method may be used as appropriate.

The feature value 220 may include any data that represent a feature of a thermosetting epoxy resin composition. For example, the feature value 220 may include a blend raw material and a blend ratio related to a thermosetting epoxy resin composition. For example, the feature value 220 may include at least one of a molecular fingerprint and a molecular descriptor. Such a molecular descriptor is, for example, MaxPartialCharge, MinPartialCharge, MaxEStateIndex, MinEStateIndex, QED, MolMR, MolWt, ExactMolWt, HeavyAtomCount, HeavyAtomMolWt, NHOHCount, NOCount, NumHAcceptors, NumHDonors, NumHeteroatoms, NumRotatableBonds, NumValenceElectrons, NumAmideBonds, NumAromaticRings, NumSaturatedRings, NumAliphaticRings, NumAromaticHeterocycles, NumSaturatedHeterocycles, NumAliphaticHeterocycles, NumAromaticCarbocycles, NumSaturatedCarbocycles, NumAliphaticCarbocycles, RingCount, FractionCSP3, NumSpiroAtoms, NumBridgeheadAtoms, TPSA, LabuteASA, PEOE_VSA1 to PEOE_VSA14, SMR_VSA1 to SMR_VSA10, SlogP_VSA1 to SlogP_VSA12, EState_VSA1 to EState_VSA11, VSA_EState1 to VSA_EState10, BCUT2D, BalabanJ, BertzCT, Ipc, HallKierAlpha, Kappa1-Kappa3, Phi, Chi0, Chi1, Chi0n to Chi4n, or Chi0v to Chi4v. Other useful descriptors generated can be appropriately used.

The actual data 100, the feature values 210 and 220, and the predictive models 400 may be stored in an external device different from the information processor 10. In such a case, the information processor 10 may have an external communication interface. The communication interface may be an interface for wired communication or wireless communication. For wired communication, the communication interface is, for example, a LAN interface or USB. For wireless communication, the communication interface is, for example, an interface according to mobile communication standards, such as LTE, 4G, or 5G, or an interface according to near field communication, such as Bluetooth (registered trademark). The communication interface can receive data used for the operation of the information processor 10 and can transmit data obtained by the operation of the information processor 10.

### (Operation of Information Processor)

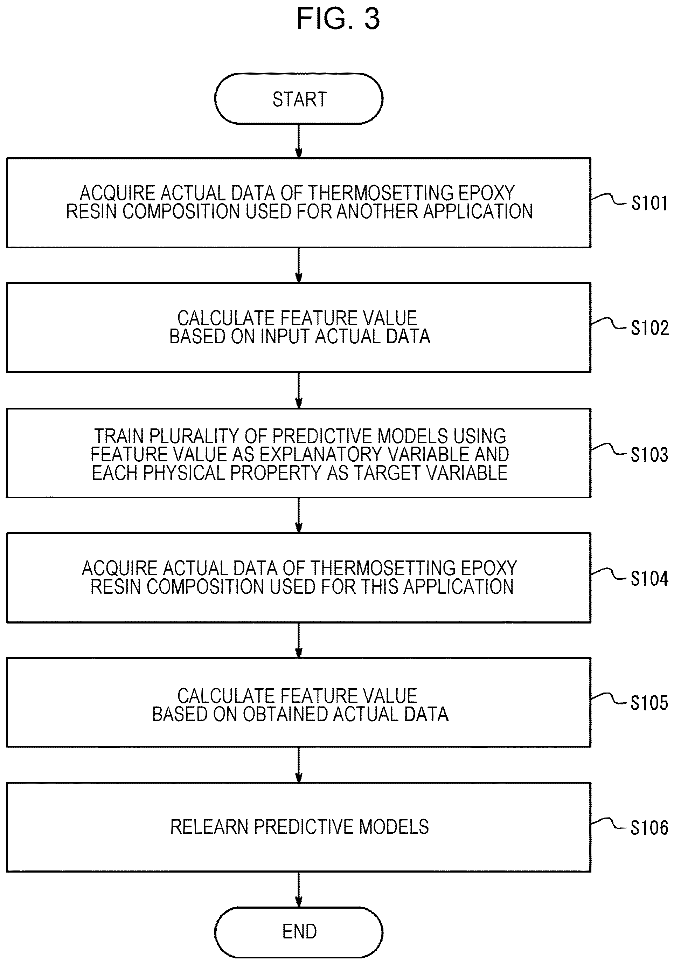

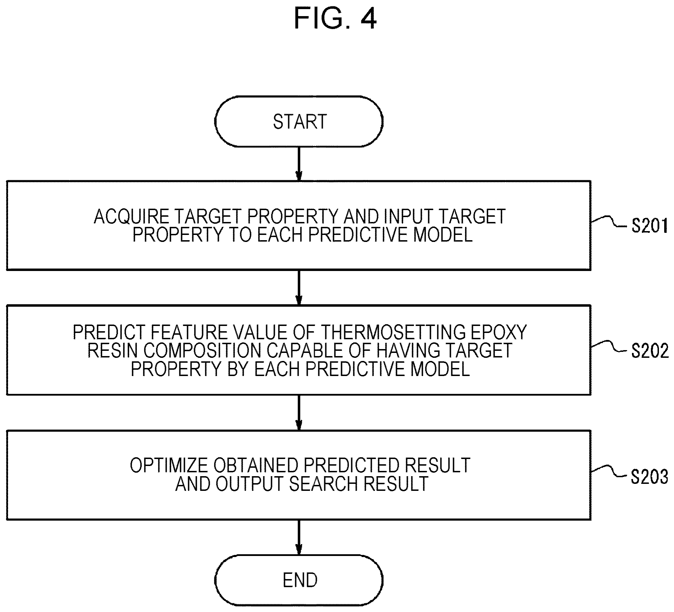

The operation of the information processor 10 according to the present embodiment is described below with reference to Figs. 3 and 4. Fig. 3 is a flow chart of an example of the learning process and the relearning process executed by the information processor 10 according to the present embodiment. Fig. 4 is a flow chart of the search process executed by the information processor 10 according to the present embodiment. First, an example of the learning process and the relearning process executed by the information processor 10 is described with reference to Fig. 3.

Step S101: The control unit 11 of the information processor 10 acquires actual data 110 of a thermosetting epoxy resin composition to be used for another application. The actual data 110 can be acquired by any method. For example, the control unit 11 may receive an input of actual data from the user through the input unit 13 to acquire the actual data 110. For example, the control unit 11 may also acquire the actual data 110 from an external device storing the actual data 110 via a communication interface.

Step S102: The control unit 11 calculates the feature value 210 on the basis of the input actual data 110. More specifically, the control unit 11 calculates the feature value 210 on the basis of a blend raw material and a blend ratio of a thermosetting epoxy resin composition, a polymer composition related to the blend raw material, and a structural formula related to the blend raw material, which are included in the actual data 110. To calculate the feature value 210, the control unit 11 may appropriately refer to a database, a library, or the like. In such a case, the database, the library, and the like may be stored in the storage unit 12.

Step S103: The control unit 11 trains a plurality of predictive models 400 (first to N-th predictive models) using the calculated feature value 210 as an explanatory variable and each physical property as a target variable. Each predictive model is, for example, but not limited to, a support vector machine, a linear model, or a nonlinear model. For example, each predictive model 400 may be a model trained on the basis of a multilayer perceptron consisting of an input layer, a hidden layer, and an output layer. Alternatively, each predictive model 400 may be a model trained on the basis of a machine learning algorithm, such as a convolutional neural network (CNN), a recurrent neural network (RNN), or another deep learning.

Step S104: The control unit 11 acquires the actual data 120 of a thermosetting epoxy resin composition used for this application. The actual data 120 can be acquired by any method. For example, the control unit 11 may receive an input of actual data from the user through the input unit 13 to acquire the actual data 120. For example, the control unit 11 may also acquire the actual data 120 from an external device storing the actual data 120 via a communication interface. The actual data 120 may be smaller in amount than the actual data 110.

Step S105: The control unit 11 calculates the feature value 220 on the basis of the input actual data 120. More specifically, the control unit 11 calculates the feature value 220 on the basis of a blend raw material and a blend ratio of a thermosetting epoxy resin composition, a polymer composition related to the blend raw material, and a structural formula related to the blend raw material, which are included in the actual data 120. To calculate the feature value 220, the control unit 11 may appropriately refer to a database, a library, or the like. In such a case, the database, the library, and the like may be stored in the storage unit 12.

Step S106: The control unit 11 relearns the predictive models 400 (the first to N-th predictive models) trained in the step S103 using the calculated feature value 220 as an explanatory variable and each physical property as a target variable. In this manner, the predictive models 400 according to the present embodiment are constructed.

The accuracy of the predictive models 400 constructed by these processes may be verified on the basis of known data. When the accuracy has been verified in a practical range, a search process of a thermosetting epoxy resin composition may be performed using the predictive models 400.

Next, an example of a search process for a thermosetting epoxy resin composition executed by the information processor 10 is described with reference to Fig. 4. As an overview, the information processor 10 searches for a thermosetting epoxy resin composition with a desired balance of physical properties by inverse analysis using a plurality of predictive models 400.

Step S201: The control unit 11 of the information processor 10 acquires a physical property (hereinafter referred to as a target property) related to a desired thermosetting epoxy resin composition and inputs the target property to each of the predictive models 400 (first to N-th predictive models). For example, the control unit 11 receives an input of the target property from the user through the input unit 13 to acquire the target property.

Step S202: The control unit 11 predicts, by each predictive model 400, the feature value of a thermosetting epoxy resin composition capable of having the target property acquired in the step S201.

Step S203: The control unit 11 optimizes the result predicted in the step S202 and outputs search results from the output unit 14. For example, the control unit 11 outputs as search results from the output unit 14 a blend ratio related to a thermosetting epoxy resin composition with a desired balance of physical properties, a polymer composition related to a blend raw material, and a structural formula related to the blend raw material. Alternatively, the control unit 11 may output as a search result from the output unit 14 a feature value related to at least one thermosetting epoxy resin composition with a desired balance of physical properties.

Here, the evaluation function can be maximized or minimized by optimization processing, a steepest descent method, Bayesian optimization, Gaussian process optimization, a Python library, such as GPyOpt, Optuna, or HyperOpt, using a mechanism thereof, a genetic algorithm, or the like. However, the present invention is not limited to these methods, and one or a plurality of methods suitable for an objective to be optimized can be selected.

As described above, in the present embodiment, the predictive models 400 are trained on the basis of the actual data 100 related to a thermosetting epoxy resin composition. A thermosetting epoxy resin composition with a desired balance of physical properties can be searched for by inverse analysis using the plurality of predictive models 400. For example, a thermosetting epoxy resin composition with desired strength and heat resistance can be easily searched.

A method of searching for a thermosetting epoxy resin composition on the basis of the experience and intuition of a person in charge without using the search method according to the present embodiment is also conceivable. In such a case, a preliminary experiment related to a known thermosetting epoxy resin composition and an unknown thermosetting epoxy resin composition is performed, and the experimental results are subjected to least-squares regression calculation to find correlations of given conditions and physical properties on the basis of the experience and intuition of a person in charge. After finding the correlations, a synthetic experiment of several thermosetting epoxy resin compositions related to search candidates is performed. The experimental results are further subjected to least-squares regression calculation. These can also be repeated to search for a desired thermosetting epoxy resin composition. However, such a method depends on the experience and intuition of a person in charge, requires enormous number of man-hours for the preliminary experiment and experiment, and typically requires several months to perform one optimal composition search. In contrast, the present embodiment can search for desired thermosetting epoxy resin compositions in parallel and in a short time in the information processor 10 on the basis of the predictive models 400. This can greatly reduce the number of development steps.

In the present embodiment, the actual data 100 include the actual data 110 for another application and the actual data 120 for the predetermined application, which are used for the learning process and the relearning process of the predictive models 400. Thus, teaching data in the learning process are in a wider range than in the predetermined application, which can prevent the accuracy from being lowered by extrapolation. Furthermore, teaching data in the relearning process are limited for the present application. This allows a highly accurate predictive model to be trained in the search for a thermosetting epoxy resin composition for the predetermined application. Although the learning process and the relearning process are performed in the present embodiment, if the accuracy is in a practical range, only the learning process may be performed, and the relearning process may be omitted. Without the relearning process, either actual data for the predetermined application or actual data for another application may be used in the learning process. This allows a predictive model to be trained in a shorter time.

Although the predetermined application is for a composite material in the present embodiment, the present invention is not limited thereto. For example, the predetermined application may be an electronic material, a paint, a civil engineering and building material, an adhesive agent, a flame retardant, or a composite material.

In the present embodiment, the feature value may include at least one of a molecular fingerprint and a molecular descriptor. Since the molecular fingerprint or the molecular descriptor can indicate a feature of a thermosetting epoxy resin composition, the feature value can be used as an explanatory variable to improve the accuracy of the predictive models 400.

In the present embodiment, the feature value may include Log P related to a thermosetting epoxy resin composition. Log P is highly correlated with the bending tensile strength, the fracture toughness, and the glass transition temperature of a thermosetting epoxy resin composition and can be used as an explanatory variable to improve the accuracy of the predictive models 400.

Tables 1 and 2 show results obtained when Log P was not used as an explanatory variable and when Log P was used as an explanatory variable in the learning of the predictive models 400. eXtreme Gradient Boosting (XGBoost) was employed as a learning model related to the glass transition temperature. On the other hand, Support Vector Machine (a kernel method) (Kernel SVM) was employed as a learning model related to bending tensile strength and fracture toughness. In Tables 1 and 2, R² denotes a coefficient of determination, and R² closer to 1 indicates a model with higher accuracy. In Tables 1 and 2, RMSE is a measure obtained by summing the root mean square of prediction errors, and RMSE closer to 0 indicates a model with higher performance.

**[Table 1]**

| Prediction accuracy results when Log P was not used as an explanatory variable | | | |
|---|---|---|---|
| | Model | R² | RMSE |
| Glass transition temperature (Tg E') | XGBoost | 0.55 | 13.5 |
| Fracture toughness (FT) | Kernel SVM | 0.86 | 0.29 |
| Tensile strength (TS) | Kernel SVM | 0.79 | 2.6 |

**[Table 2]**

| Prediction accuracy results when Log P was used as an explanatory variable | | | |
|---|---|---|---|
| | Model | R² | RMSE |
| Glass transition temperature (Tg E') | XGBoost | 0.79 | 9.2 |
| Fracture toughness (FT) | Kernel SVM | 0.95 | 0.18 |
| Tensile strength (TS) | Kernel SVM | 0.84 | 2.3 |

As shown in Tables 1 and 2, R² and RMSE of the learning model are better when Log P is used as an explanatory variable than when Log P is not used as an explanatory variable. Thus, Log P can be used as an explanatory variable to improve the accuracy of the predictive models 400. As a result, the learning model can have R² of more than 0.60 in all the target variables, and a thermosetting epoxy resin composition with a desired balance of physical properties can be designed.

Although the present disclosure has been described on the basis of the drawings and the examples, it should be noted that a person skilled in the art can easily make various changes and modifications on the basis of the present disclosure. Thus, it should be noted that these changes and modifications fall within the scope of the present disclosure. For example, functions or the like included in each means, each step, or the like can be rearranged so as not to be logically inconsistent, and a plurality of means, steps, or the like can be combined into one or divided.

### Reference Signs List

- 10: information processor
- 11: control unit
- 12: storage unit
- 13: input unit
- 14: output unit
- 100: actual data
- 110: actual data for another application
- 120: actual data for predetermined application
- 210, 220: feature value
- 310: learning process
- 320: relearning process
- 400: predictive model

## Claims

1. A method of searching for a thermosetting epoxy resin composition executed by an information processor (10), the method comprising the steps of:
calculating a feature value (210, 220) on the basis of actual data (100) related to a thermosetting epoxy resin composition, using the feature value (210, 220) as an explanatory variable of a plurality of predictive models (400), and training the plurality of predictive models (400) each corresponding to a target variable using the actual data, wherein the feature value (210, 220) includes Log P related to a thermosetting epoxy resin composition; and
searching for a thermosetting epoxy resin composition with a desired balance of physical properties by inverse analysis using the plurality of predictive models (400),
wherein the actual data (100) include a blend raw material and a blend ratio of the thermosetting epoxy resin composition, a polymer composition related to the blend raw material, and a structural formula related to the blend raw material, and
the target variables include tensile strength, fracture toughness, and a glass transition temperature.

2. The method of searching for a thermosetting epoxy resin composition according to Claim 1, wherein
the plurality of predictive models (400) include a predictive model to which the tensile strength is input, a predictive model to which the fracture toughness is input, and a predictive model to which the glass transition temperature is input.

3. The method of searching for a thermosetting epoxy resin composition according to Claim 1, wherein the feature value (210, 220) further includes at least one of a molecular fingerprint and a molecular descriptor.

4. The method of searching for a thermosetting epoxy resin composition according to any one of Claims 1 to 3, wherein
the actual data (100) include actual data of a thermosetting epoxy resin composition used for a predetermined application (120) and actual data of a thermosetting epoxy resin composition used for an application other than the predetermined application (110), and
the step of training the plurality of predictive models (400) includes
training the plurality of predictive models (400) using the actual data of the thermosetting epoxy resin composition used for the application other than the predetermined application (110) and then relearning the plurality of predictive models (400) using the actual data of the thermosetting epoxy resin composition used for the predetermined application (120).

5. An information processor (10) that includes a control unit (11) configured to search for a thermosetting epoxy resin composition, wherein
the control unit (11) is configured to
calculate a feature value (210, 220) on the basis of actual data (100) related to a thermosetting epoxy resin composition, use the feature value as an explanatory variable of a plurality of predictive models, and train the plurality of predictive models each corresponding to a target variable using the actual data (100), wherein the feature value includes Log P related to a thermosetting epoxy resin composition, and
searche for a thermosetting epoxy resin composition with a desired balance of physical properties by inverse analysis using the plurality of predictive models (400),
wherein the actual data (100) include a blend raw material and a blend ratio of the thermosetting epoxy resin composition, a polymer composition related to the blend raw material, and a structural formula related to the blend raw material, and
the target variables include tensile strength, fracture toughness, and a glass transition temperature.

6. A non-transitory computer readable medium storing a program of searching for a thermosetting epoxy resin composition, the program comprising instructions that when executed:
cause a computer
to calculate a feature value (210, 220) on the basis of the actual data (100) related to a thermosetting epoxy resin composition, to use the feature value (210, 220) as an explanatory variable of a plurality of predictive models (400), and to train the plurality of predictive models (400) each corresponding to a target variable using the actual data, wherein the feature value (210, 220) includes Log P related to a thermosetting epoxy resin composition, and
to search for a thermosetting epoxy resin composition with a desired balance of physical properties by inverse analysis using the plurality of predictive models (400),
wherein the actual data (100) include a polymer composition, a reaction solvent, and a reaction parameter related to the thermosetting epoxy resin composition,
the actual data include a blend raw material and a blend ratio of the thermosetting epoxy resin composition, a polymer composition related to the blend raw material, and a structural formula related to the blend raw material, and
the target variables include tensile strength, fracture toughness, and a glass transition temperature.

## Patentansprüche

1. Verfahren zum Suchen einer wärmehärtbaren Epoxidharzzusammensetzung, das von einem Informationsprozessor (10) ausgeführt wird, wobei das Verfahren die Schritte umfasst:
Berechnen eines Merkmalswerts (210, 220) auf der Basis von tatsächlichen Daten (100) bezogen auf eine wärmehärtbare Epoxidharzzusammensetzung, Verwenden des Merkmalswerts (210, 220) als erklärende Variable einer Vielzahl von Vorhersagemodellen (400) und Trainieren der Vielzahl von Vorhersagemodellen (400), die jeweils einer Zielvariablen entsprechen, mittels der tatsächlichen Daten, wobei der Merkmalswert (210, 220) Log P, bezogen auf eine wärmehärtbare Epoxidharzzusammensetzung, einschließt; und
Suchen nach einer wärmehärtbaren Epoxidharzzusammensetzung mit einem gewünschten Gleichgewicht der physikalischen Eigenschaften durch inverse Analyse mittels der Vielzahl von Vorhersagemodellen (400),
wobei die tatsächlichen Daten (100) eine Mischung aus Rohmaterialien und ein Mischungsverhältnis der wärmehärtbaren Epoxidharzzusammensetzung, eine Polymerzusammensetzung, bezogen auf die Mischung aus Rohmaterialien, und eine Strukturformel, bezogen auf die Mischung aus Rohmaterialien, einschließen, und
die Zielvariablen Zugfestigkeit, Bruchzähigkeit und eine Glasübergangstemperatur einschließen.

2. Verfahren zum Suchen einer wärmehärtbaren Epoxidharzzusammensetzung nach Anspruch 1, wobei
die Vielzahl von Vorhersagemodellen (400) ein Vorhersagemodell einschließt, in das die Zugfestigkeit eingegeben wird, ein Vorhersagemodell, in das die Bruchzähigkeit eingegeben wird, und ein Vorhersagemodell, in das die Glasübergangstemperatur eingegeben wird.

3. Verfahren zum Suchen einer wärmehärtbaren Epoxidharzzusammensetzung nach Anspruch 1, wobei der Merkmalswert (210, 220) ferner mindestens einen molekularen Fingerabdruck und einen molekularen Deskriptor einschließt.

4. Verfahren zum Suchen einer wärmehärtbaren Epoxidharzzusammensetzung nach einem der Ansprüche 1 bis 3, wobei
die tatsächlichen Daten (100) tatsächliche Daten einer wärmehärtbaren Epoxidharzzusammensetzung, die für eine vorbestimmte Anwendung (120) verwendet wird, und tatsächliche Daten einer wärmehärtbaren Epoxidharzzusammensetzung, die für eine andere als die vorbestimmte Anwendung (110) verwendet wird, einschließen, und
der Schritt des Trainierens der Vielzahl von Vorhersagemodellen (400) einschließt
Trainieren der Vielzahl von Vorhersagemodellen (400) mittels der tatsächlichen Daten der wärmehärtbaren Epoxidharzzusammensetzung, die für die andere als die vorbestimmte Anwendung (110) verwendet wird, und dann Neulernen der Vielzahl von Vorhersagemodellen (400) mittels der tatsächlichen Daten der wärmehärtbaren Epoxidharzzusammensetzung, die für die vorbestimmte Anwendung (120) verwendet wird.

5. Informationsprozessor (10), der eine Steuereinheit (11) einschließt, die konfiguriert ist, um nach einer wärmehärtbaren Epoxidharzzusammensetzung zu suchen, wobei
die Steuereinheit (11) konfiguriert ist zum
Berechnen eines Merkmalswerts (210, 220) auf der Basis von tatsächlichen Daten (100) bezogen auf eine wärmehärtbare Epoxidharzzusammensetzung, Verwenden des Merkmalswerts als erklärende Variable einer Vielzahl von Vorhersagemodellen und Trainieren der Vielzahl von Vorhersagemodellen, die jeweils einer Zielvariablen entsprechen, mittels der tatsächlichen Daten (100), wobei der Merkmalswert Log P, bezogen auf eine wärmehärtbare Epoxidharzzusammensetzung, einschließt, und
Suchen nach einer wärmehärtbaren Epoxidharzzusammensetzung mit einem gewünschten Gleichgewicht der physikalischen Eigenschaften durch inverse Analyse mittels der Vielzahl von Vorhersagemodellen (400),
wobei die tatsächlichen Daten (100) eine Mischung aus Rohmaterialien und ein Mischungsverhältnis der wärmehärtbaren Epoxidharzzusammensetzung, eine Polymerzusammensetzung, bezogen auf die Mischung aus Rohmaterialien, und eine Strukturformel, bezogen auf die Mischung aus Rohmaterialien, einschließen, und
die Zielvariablen Zugfestigkeit, Bruchzähigkeit und eine Glasübergangstemperatur einschließen.

6. Nicht-transitorisches, computerlesbares Medium, das ein Programm zum Suchen nach einer wärmehärtbaren Epoxidharzzusammensetzung speichert, wobei das Programm Anweisungen umfasst, die bei Ausführung
einen Computer veranlassen
einen Merkmalswert (210, 220) auf der Basis von tatsächlichen Daten (100) bezogen auf eine wärmehärtbare Epoxidharzzusammensetzung zu berechnen, den Merkmalswert (210, 220) als erklärende Variable einer Vielzahl von Vorhersagemodellen (400) zu verwenden und die Vielzahl von Vorhersagemodellen (400), die jeweils einer Zielvariablen entsprechen, mittels der tatsächlichen Daten zu trainieren, wobei der Merkmalswert (210, 220) Log P, bezogen auf eine wärmehärtbare Epoxidharzzusammensetzung, einschließt, und
um eine wärmehärtbare Epoxidharzzusammensetzung mit einem gewünschten Gleichgewicht der physikalischen Eigenschaften durch inverse Analyse mittels der Vielzahl von Vorhersagemodellen (400) zu suchen,
wobei die tatsächlichen Daten (100) eine Polymerzusammensetzung, ein Reaktionslösungsmittel und einen Reaktionsparameter, die sich auf die wärmehärtbare Epoxidharzzusammensetzung beziehen, einschließen,
die tatsächlichen Daten eine Mischung aus Rohmaterialien und ein Mischungsverhältnis der wärmehärtbaren Epoxidharzzusammensetzung, eine Polymerzusammensetzung, bezogen auf die Mischung aus Rohmaterialien, und eine Strukturformel, bezogen auf die Mischung aus Rohmaterialien, einschließen, und
die Zielvariablen Zugfestigkeit, Bruchzähigkeit und eine Glasübergangstemperatur einschließen.

## Revendications

1. Procédé de recherche d'une composition de résine époxy thermodurcissable exécuté par un processeur d'informations (10), le procédé comprenant les étapes suivantes :
calculer une valeur caractéristique (210, 220) sur la base de données réelles (100) liées à une composition de résine époxy thermodurcissable, utiliser la valeur caractéristique (210, 220) comme variable explicative d'une pluralité de modèles prédictifs (400), et entraîner la pluralité de modèles prédictifs (400) correspondant chacun à une variable cible en utilisant les données réelles, dans lequel la valeur caractéristique (210, 220) comprend le Log P lié à une composition de résine époxy thermodurcissable ; et
rechercher une composition de résine époxy thermodurcissable présentant un équilibre souhaité de propriétés physiques par analyse inverse à l'aide d'une pluralité de modèles prédictifs (400),
dans lequel les données réelles (100) incluent une matière première de mélange et un rapport de mélange de la composition de résine époxy thermodurcissable, une composition polymère liée à la matière première de mélange, et une formule structurale liée à la matière première de mélange, et
les variables cibles incluent une résistance à la traction, une ténacité à la rupture et une température de transition vitreuse.

2. Procédé de recherche d'une composition de résine époxy thermodurcissable selon la revendication 1, dans lequel
la pluralité de modèles prédictifs (400) inclut un modèle prédictif dans lequel la résistance à la traction est saisie, un modèle prédictif dans lequel la ténacité à la rupture est saisie, et un modèle prédictif dans lequel la température de transition vitreuse est saisie.

3. Procédé de recherche d'une composition de résine époxy thermodurcissable selon la revendication 1, dans lequel la valeur caractéristique (210, 220) inclut en outre au moins un parmi une empreinte moléculaire et un descripteur moléculaire.

4. Procédé de recherche d'une composition de résine époxy thermodurcissable selon l'une quelconque des revendications 1 à 3, dans lequel
les données réelles (100) incluent les données réelles d'une composition de résine époxy thermodurcissable utilisée pour une application prédéterminée (120) et les données réelles d'une composition de résine époxy thermodurcissable utilisée pour une application autre que l'application prédéterminée (110), et
l'étape consistant à entraîner la pluralité de modèles prédictifs (400) inclut
entraîner la pluralité de modèles prédictifs (400) à l'aide des données réelles de la composition de résine époxy thermodurcissable utilisée pour l'application autre que l'application prédéterminée (110), puis réapprendre la pluralité de modèles prédictifs (400) à l'aide des données réelles de la composition de résine époxy thermodurcissable utilisée pour l'application prédéterminée (120).

5. Processeur d'informations (10) incluant une unité de commande (11) configurée pour rechercher une composition de résine époxy thermodurcissable, dans lequel
l'unité de commande (11) est configurée pour
calculer une valeur caractéristique (210, 220) sur la base de données réelles (100) liées à une composition de résine époxy thermodurcissable, utiliser la valeur caractéristique comme variable explicative d'une pluralité de modèles prédictifs, et entraîner la pluralité de modèles prédictifs correspondant chacun à une variable cible en utilisant les données réelles (100), dans lequel la valeur caractéristique comprend le Log P lié à une composition de résine époxy thermodurcissable, et
rechercher une composition de résine époxy thermodurcissable présentant un équilibre souhaité de propriétés physiques par analyse inverse à l'aide d'une pluralité de modèles prédictifs (400),
dans lequel les données réelles (100) incluent une matière première de mélange et un rapport de mélange de la composition de résine époxy thermodurcissable, une composition polymère liée à la matière première de mélange, et une formule structurale liée à la matière première de mélange, et
les variables cibles incluent une résistance à la traction, une ténacité à la rupture et une température de transition vitreuse.

6. Support lisible par ordinateur non transitoire stockant un programme de recherche d'une composition de résine époxy thermodurcissable, le programme comprenant des instructions qui, lorsqu'elles sont exécutées :
amener un ordinateur à
calculer une valeur caractéristique (210, 220) sur la base des données réelles (100) liées à une composition de résine époxy thermodurcissable, utiliser la valeur caractéristique (210, 220) comme variable explicative d'une pluralité de modèles prédictifs (400), et entraîner la pluralité de modèles prédictifs (400) correspondant chacun à une variable cible en utilisant les données réelles, dans lequel la valeur caractéristique (210, 220) comprend le Log P lié à une composition de résine époxy thermodurcissable, et
rechercher une composition de résine époxy thermodurcissable présentant un équilibre souhaité de propriétés physiques par analyse inverse à l'aide d'une pluralité de modèles prédictifs (400),
dans lequel les données réelles (100) incluent une composition polymère, un solvant réactionnel et un paramètre réactionnel lié à la composition de résine époxy thermodurcissable,
les données réelles incluent une matière première de mélange et un rapport de mélange de la composition de résine époxy thermodurcissable, une composition polymère liée à la matière première de mélange, et une formule structurale liée à la matière première de mélange, et
les variables cibles incluent une résistance à la traction, une ténacité à la rupture et une température de transition vitreuse.
